Europäisches Patentamt

European Patent Office

Office européen des brevets

(19)

(11) Numéro de publication: **0 006 059**
**A1**

(12) **DEMANDE DE BREVET EUROPEEN**

(21) Numéro de dépôt: **79400338.4**

(22) Date de dépôt: **29.05.79**

(51) Int. Cl.²: **A 61 K 35/78**

(30) Priorité: **31.05.78 GB 2561978**

(43) Date de publication de la demande:
**12.12.79 Bulletin 79/25**

(84) Etats Contractants Désignés:
**AT BE CH DE FR GB IT LU NL SE**

(71) Demandeur: **Société Anonyme dite: LABORATOIRES DEBAT**
**60 rue de Monceau**
**F-75008 Paris(FR)**

(72) Inventeur: **Debat, Jacques**
**3 rue du Pierrier**
**F-92210 Saint-Cloud(FR)**

(72) Inventeur: **Lemoine, Jean**
**7 Sente de la Portaille**
**F-92380 Garches(FR)**

(72) Inventeur: **Longuet, Monique**
**11 rue des Grands Jardins**
**F-78770 Auteuil Le Roi(FR)**

(74) Mandataire: **Clisci, Serge et al,**
**CABINET BEAU DE LOMENIE 55 rue d'Amsterdam**
**F-75008 Paris(FR)**

(54) Extrait de Globularia, son procédé de préparation et son application en thérapeutique.

(57) 1°) La présente invention concerne un procédé d'extraction de *Globularia alypum*, *Globularia vulgaris* et des espèces apparentées appartenant à la famille des Globulariacées selon lequel on traite de préférence la plante entière, les tiges ou les feuilles au moyen de deux solvants d'extraction de polarité différente.

2°) L'extrait obtenu selon ce procédé est utile en thérapeutique humaine et vétérinaire notamment dans le traitement de la brucellose.

EP 0 006 059 A1

La présente invention a trait à un extrait de plante du genre Globularia tel que Globularia alypum, Globularia vulgaris et les espèces apparentées appartenant à la famille des Globulariacées, qui est utile en tant qu'agent antibrucella, bactériostatique et anti-inflammatoire.

Cet extrait est obtenu à partir de la plante entière ou d'une partie de ladite plante au moyen de deux solvants d'extraction de polarités différentes.

La plante Globularia alypum, qui appartient à la famille des Globulariacées, pousse principalement dans le bassin méditerranéen. Cette plante a été décrite par HEGNAUER dans "Chemotaxonomie der Pflanzen", vol.4, pages 207, 210 (1966) de même que l'espèce Globularia vulgaris et les espèces apparentées de la famille des Globulariacées.

Dans le passé l'espèce Globularia alypum a été utilisée ou proposée en médecine populaire pour un grand nombre de maladies. En particulier les tisanes (infusions aqueuses) de fleurs de Globularia alypum ont été proposées en tant qu'agents laxatifs, diurétiques, et antibrucella, et les extraits aqueux de feuilles ont été proposés dans le traitement des rhumatismes, de la goutte, de la typhoïde, des fièvres inttermitentes, comme agents antiseptiques urinaires et laxatifs.

Plus récemment, il a été décrit par G. CALDES et al., dans Planta Medica 27, 72-76 (1975) que des extraits obtenus à partir de la plante entière de Globularia alypum par extraction avec de l'eau froide ou bouillante puis lyophilisation sont (i) inactifs en tant qu'agents bactériostatiques (vis à vis de <u>Staphylococcus</u> <u>aureus</u> et <u>Streptococcus</u> <u>faecium</u>) et antimalaria (vis à vis de <u>Plasmodium</u> <u>berghi</u>, et (ii) actifs en tant qu'agents antileucémiques.

Compte tenu des utilisations nombreuses et divergentes qui ont été proposées dans le passé pour les extraits de <u>Globularia</u> <u>alypum</u>, des études complémentaires ont été effectuées. Ces études ont à présent conduit à un nouveau procédé d'extraction de <u>Globularia</u> <u>alypum</u>, de <u>Globularia</u> <u>vulgaris</u> et d'espèces apparentées de la famille des Globulariacées, qui de façon surprenante donnent un extrait qui (i) est bien plus actif en tant qu'agent antibrucella que les extraits connus antérieurement, (ii) présente une activité bactériostatique vis à vis des bactéries Gram (+) tel que le <u>Staphylococcus</u> <u>aureus</u>, et (iii) est également utile en tant qu'agent anti-inflammatoire.

Selon l'invention on propose un procédé d'extraction de la plante entière (c'est à dire les tiges, les feuilles, les sommités fleuries, les fruits et les racines) ou une partie de la plante, telles que par exemple les tiges, les feuilles et les tiges feuillées (i.e. les tiges et les feuilles) au moyen de deux solvants d'extraction de polarité différente.

Selon l'invention on préconise également une composition thérapeutique utile notamment en médecine humaine et vétérinaire, en particulier dans le traitement de la brucellose, caractérisée en ce qu'elle renferme, en association avec un excipient physiologiquement acceptable, une quantité pharmaceutiquement active d'un extrait de <u>Globularia</u>.

3

0006059

Le procédé selon l'invention pour la préparation d'un extrait de <u>Globularia</u> au moyen de deux solvants de polarité différente, est caractérisé en ce que successivement :

(a) on soumet 10 à 120 g de <u>Globularia</u> secs et broyés choisis parmi l'ensemble constitué par la plante entière ou une partie de celle-ci, de <u>Globularia alypum</u>, <u>Globularia vulgaris</u> et d'espèces apparentées de la famille des Globulariacées, à une première extraction avec 1 litre d'un solvant choisi parmi l'eau, les alcools, les cétones, les éthers, les esters, les hydrocarbures, les hydrocarbures halogénés et leurs mélanges ;

(b) on élimine l'insoluble par filtration et soumet le filtrat à un traitement choisi parmi l'évaporation à sec sous pression réduite, la nébulisation et la lyophilisation ;

(c) on soumet le produit sec ainsi obtenu, à une seconde extraction avec un solvant différent du solvant d'extraction du stade (a) par sa polarité et choisi parmi l'ensemble constitué par les mélanges eau-alcool, les alcools, les cétones, le chlorure de méthylène, le chloroforme et leurs mélanges ;

(d) on élimine l'insoluble par filtration et évapore le filtrat à sec sous pression réduite ; et, si nécessaire,

(e) on soumet le produit sec obtenu au stade (d) à une purification, notamment à une filtration moléculaire par chromatographie avec un support qui absorbe relativement mieux les substances de bas poids moléculaire que les substances de haut poids moléculaire, de façon à recueillir une fraction renfermant essentiellement la plupart des substances de haut poids moléculaire contenues dans le produit sec du stade (d).

L'extraction du stade (a) peut être effectuée en utilisant pour 1 litre de solvant 10 à 120 g de plante (ou une partie de celle-ci) séchée et broyée. Le matériau de départ préféré est la plante entière, les tiges, les feuilles et les tiges feuillées. Le matériau le plus intéressant est les tiges feuillées.

Parmi les solvants du stade (a) qui peuvent être utilisés on peut notamment mentionner l'eau, les alcools (tels que les méthanol, éthanol, propanol, isopropanol), les cétones (tels que les acétone, méthyléthylcétone, méthylpropylcétone), les éthers (tels que les éthers diméthylique, diéthylique, diisopropylique, les esters (tels que l'acétate d'éthyle), les hydrocarbures (tels que les pentane, hexane, cyclopentane, cyclohexane, éther de pétrole, benzène), les hydrocarbures halogénés (tels que les chloroforme, chlorure de méthylène) et leurs mélanges.

Le stade (b) a pour but de recueillir un produit sec à partir du filtrat. La lyophilisation et la nébulisation (i.e. séchage par projection) sont utilisées quand l'extraction du stade (a) a été réalisée avec de l'eau. L'évaporation à sec sous pression réduite est utilisée quand le solvant du stade (a) est un solvant organique ou un mélange eau-solvant organique. L'évaporation sous pression réduite puis la lyophilisation ou la nébulisation peuvent également être mises en oeuvre successivement quand le solvant du stade (a) est un mélange d'eau et de solvant organique.

Pour l'extraction du stade (c) on utilise de préférence 50 à 300 ml de solvant par gramme de produit (b) à traiter, les solvants préférés étant le méthanol, l'éthanol et leurs mélanges avec l'eau.

Au stade (d) une quantité relativement importante de substances insolubles, et non actives vis à vis des _Brucella_, est écartée. D'une manière générale, quand le solvant du stade (c) est moins polaire que celui du stade (a), le stade (c) permet d'éliminer environ 15 à 35 % en poids sec d'insoluble par rapport au poids du produit sec (b) à traiter.

Le stade (e) est de préférence mis en oeuvre avec comme éluant de l'eau, des alcools micibles avec l'eau, l'acétone ou leurs mélanges. Le support chromatographique peut être soit de nature ionique, soit de nature non ionique.

Le meilleur mode pour mettre en oeuvre le procédé de préparation selon l'invention comprend :

(a) l'extraction de 10 à 120 g de tiges feuillées, séchées à l'étuve à 37°C et broyées de _Globularia_ _alypum_ avec 1 litre d'un solvant choisi parmi l'ensemble constitué par l'eau, les alcools et leurs mélanges (le solvant préféré étant l'eau distillée), à reflux pendant 15 minutes à 5 heures ;

(b) l'élimination de l'insoluble par filtration et la lyophilisation de la solution résultante ;

(c) l'extraction du produit lyophilisé ainsi obtenu, à la température ambiante (15-25°C) pendant 2 à 4 h, avec du méthanol, un mélange eau-méthanol, de l'éthanol et un mélange eau-éthanol (le solvant le plus intéressant étant ici le méthanol), en utilisant 50 à 300 ml de solvant par gramme de produit lyophilisé à extraire, et de façon avantageuse en utilisant 90 à 110 ml de solvant pour 1 g de produit lyophilisé ;

(d) l'élimination de l'insoluble par filtration et l'évaporation de la solution à sec sous pression réduite ; et,

(e) la dissolution du résidu d'évaporation ainsi obtenu , dans un solvant de préférence choisi parmi l'ensemble comprenant l'eau, le méthanol, l'éthanol et leurs mélanges, et la chromatographie sur colonne de la solution résultante sur un support convenable pour filtration moléculaire.

Le support préféré du stade (e) est un support non ionique qui est commercialisé sous le nom de SEPHADEX GH-20. On peut toutefois utiliser à la rigueur une résine échangeuse d'ions du type Amberlite XAD-2. La fraction active qui renferme de façon prédominante les substances de haut poids moléculaire est obtenue la première. Si on utilise au stade (e) 10 volumes de solvant, 3 fractions sont obtenues successivement : les trois premiers volumes constituent la Fraction I (qui renferme les substances actives), les trois volumes qui suivent constituent la Fraction II (qui est moins intéressante du point de vue thérapeutique que la Fraction I), les trois volumes qui suivent constituent la fraction III (qui est également moins intéressante que la Fraction I). En conséquence, quand on traite 1 à 5 g du résidu d'évaporation avec 1 litre de solvant, les 300 ml recueillis en premier au bas de la colonne constituent la fraction I, les 300 ml qui suivent constituent la Fraction II, puis les 300 ml qui suivent constituent la Fraction III, la durée totale pour obtenir l'ensemble des Fractions I, II, III est comprise entre 6 et 9 h (soit environ 2 à 3 h pour chaque fraction).

Si nécessaire, la Fraction I ainsi obtenue peut être évaporée à sec sous pression réduite ou lyophilisée.

On observe que, par chromatographie sur couche mince, la Fraction I donne deux taches principales de Rf 0,3 et respectivement 0,54 (voir exemples 1 et 2 ci-après).

Les exemples qui suivent nullement limitatifs ont été donnés à titre d'illustration.

EXEMPLE 1

Extrait de Globularia alypum

A/ Obtention de l'extrait total

50 g de plante entière de Globularia alypum séchés à l'étuve à 37°C et broyés sont extraits avec 1,5 litre d'eau à reflux pendant 4 heures. On écarte l'insoluble par filtration et on lyophilise la solution pour obtenir 10,2 g d'extrait total. Rendement : 20,5 % en poids par rapport à la plante de départ.

B/ Obtention des Fractions I, II et III

10 g de l'extrait total sont traités pendant 2 heures à la température ambiante (15-25°C) avec 1 litre du mélange eau-éthanol (20 : 80) v/v. L'insoluble (2 g) est écarté par filtration et la solution est évaporée à sec sous pression réduite pour donner 8 g de produit sec.

5 g du produit sec ainsi obtenu sont traités au moyen d'une résine échangeuse d'ions selon les conditions opératoires suivantes :

colonne : diamètre de 3 cm

support : 120 ml d'une résine échangeuse d'ions du type XAD-2

dépôt : 5 g d'extrait total dissous dans 20 ml d'eau distillée

éluant : 980 ml d'eau distillée (quantité totale d'eau : 1 litre)

Les 300 ml recueillis en premier au bas de la colonne sont lyophilisés pour donner la Fraction I ; les 300 ml qui suivent sont lyophilisés pour donner la Fraction II ; enfin les 300 ml suivants obtenus au bas de la colonne sont également lyophilisés pour donner la Fraction III.

La Fraction I, le produit thérapeutiquement actif selon l'invention, qui est moins adsorbée que les deux autres fractions, contient la majeure partie des substances de haut poids moléculaire qui sont présentes dans le produit
sec devant être fractionné par chromatographie.

La chromatographie sur couche mince [support :
cellulose ; phase mobile : phase supérieure du milieu de
Partridge; butanol-acide acétique-eau (4 : 1 : 5) v/v/ ;
révélation : vanilline-acide sulfurique (1 g de vanilline
pour 100 ml de $H_2SO_4$ concentré de densité 1,84)] de la
Fraction I donne 2 taches principales : une tache (rougebrun) de Rf 0,3 et une tache (bleue) de Rf 0,54.

EXEMPLE 2

Extrait de Globularia alypum
A/ Obtention de l'extrait total

100 g de tiges feuillées de Globularia alypum
séchés à l'étuve à 37°C et broyés sont extraits avec
4 litres d'eau distillée, sous reflux pendant 30 minutes.
Après refroidissement, on écarte l'insoluble par filtration
et on lyophilise le filtrat pour obtenir 42 g d'extrait
total. Rendement : 42 % en poids par·rapport au matériau de
départ.

B/ Obtention des Fractions I, II et III

3,3 g de l'extrait total sont traités, à la température ambiante (15-25°C), pendant trois heures avec 300 ml
de méthanol. On écarte l'insoluble (1,09 g en poids sec)
par filtration, et on évapore la solution à sec sous pression
réduite pour obtenir 2,21 g de produit sec.

2 g dudit produit sec en solution dans un litre de
méthanol sont soumis à une chromatographie sur colonne
(filtration moléculaire) avec une colonne ayant un diamètre
de 3 cm et garnie avec un support non ionique (120 ml de
Sephadex GH-20). Les 300 ml recueillis en premier au bas de
la colonne sont évaporés à sec sous pression réduite pour
donner la FRaction I; les 300 ml qui suivent donnent, après
évaporation à sec sous pression réduite, la Fraction II ;

enfin les 300 ml qui suivent sont évaporés à sec sous pression réduite pour donner la Fraction III.

Les rendements par rapport au produit sec chromatographié sont les suivants :

Fraction I : 3,7 % en poids

Fraction II : 69,0 % en poids

Fraction III : 15,7 % en poids

Comme indiqué dans l'exemple 1 B, la Fraction I contient, par rapport aux Fractions II et III, la majeure partie des substances de haut poids moléculaire.

La chromatographie sur couche mince de la Fraction I selon le mode opératoire donné à l'exemple 1 B, donne les deux taches principales de Rf 0,3 et Rf 0,54.

EXEMPLE 3

Extrait de Globularia vulgaris

Des tiges de Globularia vulgaris sont extraites selon le procédé décrit à l'exemple 2 avec la différence que l'extrait total est obtenu par traitement avec 4 litres du mélange eau-éthanol (85:15) v/v puis évaporation de $C_2H_5OH$ et lyophilisation. On obtient les Fractions I, II et III, la Fraction I étant le produit thérapeutiquement actif.

Les résultats des essais pharmacologiques ont été résumés ci-après :

1°/ Activité antibrucella

L'activité antibrucella des Fractions I, II et III a été comparée avec celle des extraits totaux correspondants, pris comme produits de référence, vis à vis de plusieurs souches de Brucella (les numéros des souches étant ceux du catalogue de la collection du "Service de Diagnostic des Brucelloses de l'Institut de Biologie"de Montpellier). Les concentrations minimales inhibitrices (CMI) déterminées selon la méthode des dilutions en milieu liquide ont été consignées dans le tableau I.

2°/ Activité bactériostatique

Les Fractions I des exemples 1 et 2 inhibent le développement des bactéries Gram (+) tels que notamment les Cocci, alors que, en revanche, les extraits totaux sont inactifs. En particulier les CMI vis à vis du Stapylococcus aureus Londres déterminées selon la méthode des dilutions en milieu solide (gélose) sont les suivantes :

Fraction I de l'exemple 1 : 2,50 mg/ml

Fraction I de l'exemple 2 : 1,25 mg/ml

3°/ Activité anti-inflammatoire

L'activité anti-inflammatoire des Fractions I des exemples 1 et 2 est supérieure à celle de l'aspirine.

La composition thérapeutique selon l'invention, qui renferme une quantité pharmaceutiquement efficace d'un extrait de Globularia, en association avec un excipient acceptable, peut être administré par voie orale sous la forme de comprimés, sirops, et ampoules buvables, ou par voie injectable, notamment.

TABLEAU I

CMI ( en $\mu$g/ml)

| Souches de Brucella | | Exemple 1 | | | | Exemple 2 | | | |
|---|---|---|---|---|---|---|---|---|---|
| | | Extrait Total | Fraction I | Fraction II | Fraction III | Extrait Total | Fraction I | Fraction II | Fraction III |
| Brucella melitensis type 1 | No 389 | >1000 | 100 | >2000 | 1000 | 1000 | 100 | >2000 | 1000 |
| | No 595 | 2000 | 250 | >2000 | 1000 | 2000 | 100 | >2000 | 1000 |
| | No 605 | >2000 | 250 | >2000 | 1000 | >2000 | 250 | >2000 | 1000 |
| | No 16M | >2000 | 100 | >2000 | >2000 | >2000 | 100 | >2000 | >2000 |
| Brucella melitensis type 2 | No 15 | >2000 | 250 | – | >2000 | >2000 | 100 | – | >2000 |
| | No 418 | 1000 | 4 | >2000 | >2000 | 1000 | 3,5 | >2000 | >2000 |
| | No 473 | 2000 | 100 | >2000 | 1000 | 2000 | 250 | >2000 | 1000 |
| | No 546 | >2000 | 7,2 | >2000 | >2000 | 2000 | 7,5 | >2000 | 1000 |
| Brucella abortus | No 19 | >2000 | 250 | >2000 | >2000 | >2000 | 250 | >2000 | >2000 |
| | No 112 | >2000 | 250 | – | >2000 | >2000 | 500 | >2000 | >2000 |
| | No 544 | 500 | 3,5 | >2000 | 1000 | 500 | 2,5 | >2000 | 1000 |

REVENDICATIONS
---------------

1.        Procédé de préparation d'un extrait de Globularia
utile en thérapeutique humaine et vétérinaire, en particulier dans le traitement de la brucellose, caractérisé en ce
que successivement :

(a) on soumet 10 à 120 g de Globularia secs et broyés
choisi parmi l'ensemble constitué par la plante
entière ou une partie de celle-ci, de Globularia
alypum, Globularia vulgaris et d'espèces apparentées de la famille des Globulariacées, à une première extraction avec 1 litre d'un solvant choisi
parmi l'eau, les alcools, les cétones, les éthers,
les esters, les hydrocarbures, les hydrocarbures
halogénés et leurs mélanges ;

(b) on élimine l'insoluble par filtration et soumet
le filtrat à un traitement choisi parmi l'évaporation à sec sous pression réduite, la nébulisation
et la lyophilisation ;

(c) on soumet le produit sec ainsi obtenu, à une seconde
extraction avec un solvant différent du solvant
d'extraction du stade (a) par sa polarité et choisi
parmi l'ensemble constitué par les mélanges eau-
alcool, les alcools, les cétones, le chlorure de
méthylène, le chloroforme et leurs mélanges ;

(d) on élimine l'insoluble par filtration et évapore le
filtrat à sec sous pression réduite ; et, si nécessaire,

(e) on soumet le produit sec obtenu au stade (d) à une purification notamment à une filtration moléculaire par chromatographie avec un support qui absorbe relativement mieux les substances de bas poids moléculaire, de façon à recueillir une fraction renfermant essentiellement la plupart des substances de haut poids moléculaire contenues dans le produit sec du stade (d).

2.        Procédé selon la revendication 1 caractérisé en ce que le matériau de départ est la plante entière de Globularia alypum ou de Globularia vulgaris.

3.        Procédé selon la revendication 1 caractérisé en ce que le matériau de départ est choisi parmi l'ensemble constitué par les tiges, les feuilles, les tiges feuillées, les sommités fleuries, les fruits et les racines de Globularia alypum et de Globularia vulgaris.

4.        Procédé selon la revendication 3 caractérisé en ce que le matériau de départ est constitué par les tiges et les feuilles de Globularia alypum et Globularia vulgaris.

5.        Procédé selon la revendication 1 caractérisé en ce que l'extraction du stade (a) est effectuée au reflux pendant 15 minutes à 5 heures.

6.        Procédé selon la revendication 1 caractérisé en ce que l'extraction du stade (a) est mis en oeuvre avec un solvant choisi parmi l'ensemble constitué par l'eau, le méthanol, l'éthanol et leurs mélanges.

7.        Procédé selon la revendication 1 caractérisé en ce que l'extraction du stade (c) est effectuée avec un solvant choisi parmi le méthanol, l'éthanol, et leurs mélanges avec l'eau, à la température de 15-25°C pendant 2-4 h, en utilisant 50 à 300 ml de solvant pour 1 g de produit sec à traiter.

8. Procédé selon la revendication 1 caractérisé en ce qu'il comprend les étapes suivantes :

(a) on extrait au reflux pendant 15 minutes à 5 heures, 10 à 120 g d'un matériau choisi parmi la plante entière, les tiges et les feuilles de Globularia alympum , avec 1 litre d'eau ;

(b) on écarte l'insoluble et soumetla solution à une lyophilisation ;

(c) on soumet le produit lyophilisé ainsi obtenu à une extraction à 15-25°C pendant 2-4 h, avec un solvant choisi parmi l'ensemble comprenant le méthanol, l'éthanol, et leurs mélanges avec de l'eau, en utilisant 50 à 300 ml de solvant pour 1 g de produit lyophilisé à traiter ;

(d) on écarte l'insoluble et évapore à sec la solution sous pression réduite ; et,

(e) on dissoud le résidu d'évaporation ainsi obtenu dans un solvant choisi parmi l'ensemble comprenant l'eau, le méthanol, l'éthanol et leurs mélanges, on chromatographie la solution ainsi obtenue sur une colonne garnie d'un support convenable pour filtration moléculaire, et recueille au bas de ladite colonne les 3 premiers volumes obtenus si on a déposé au sommet de la colonne 10 volumes de solvant.

9. Extrait de Globularia caractérisé en ce qu'il est obtenu à partir d'une plante choisie parmi l'ensemble comprenant les Globularia alypum, Globularia vulgaris et les espèces apparentées appartenant à la famille des Globulariacées, selon le procédé de la revendication 1.

10. Extrait de Globularia selon la revendication 9 caractérisé en ce qu'il présente par chromatographie sur couche mince [ support : cellulose ; phase mobile ; butanol-acide acétique-eau (4:1:5) v/v ; révélation : vanilline-acide sulfurique (1 g de vanilline pour 100 ml de $H_2SO_4$ concentrée de densité 1,84)] deux taches principales l'une de Rf 0,3 et l'autre de Rf 0,54.

11.     Composition thérapeutique utile notamment dans le traitement de.la brucellose caractérisée, en ce qu'elle renferme, en association avec un excipient physiologiquement acceptable, un extrait de <u>Globularia</u> selon l'une quelconque des revendications 9 et 10.

12.     Composition thérapeutique utile notamment dans le traitement de la brucellose caractérisé, en ce qu'elle renferme, en association avec un excipient physiologiquement acceptable, un extrait de <u>Globularia</u> préparé selon le procédé de l'une quelconque des revendications 1 et 8.

# RAPPORT DE RECHERCHE EUROPEENNE

Office européen des brevets

## DOCUMENTS CONSIDERES COMME PERTINENTS

| Catégorie | Citation du document avec indication, en cas de besoin, des parties pertinentes | Revendication concernée |
|---|---|---|
| XP | HELVETICA CHIMICA ACTA, vol. 62, Fasciculus 2, 07-03-1979, pages 644-6,ref.no.67,Soc.suisse de chim. Basel (CH) R.K. CHAUDHURI et O. STICHER: "Structure of Globularidin: An Unusual Iridoid Glucoside from Globularia Alypum L. * Page 644 * | 1,2,3, 6 |
| XD | PLANTA MEDICA, vol. 27, no. 1, pages 72-76 , 1975 G. CALDES et al.: "A Potential Antileukemic Substance Present In Globularia Alypum" * Article en entier * | 1-4,6, 9-12 |
| | PLANTES MEDICINALES ET PHYTO-THERAPIE, 1974, Tome VIII, no. 3, pages 174-179 P. BERNARD et al.: "Etude des Acides Aromatiques et des Com-posés Flavoniques des Feuilles de Globulaire (Globularia Alypum L.) * Article en entier * | 1,3,4, 9,10 |
| A | CHEMICAL ABSTRACTS, vol. 82, no. 9 03-03-1975, page 280, ref.54139p Columbus, Ohio, US P. BERNARD et al.: "Chromogenic Iridoid-like Heterosides in the Leaves of the Globe Daisy, Glo-bularia Alypum. & Plant. Med. Phytother. 1974 8(3) 180-7 (FR) * Abrégé * | 1 |

### CLASSEMENT DE LA DEMANDE (Int. Cl.³)

A 61 K 35/78

### DOMAINES TECHNIQUES RECHERCHES (Int. Cl.³)

A 61 K 35/78

### CATEGORIE DES DOCUMENTS CITES

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons
&: membre de la même famille, document correspondant

Le présent rapport de recherche a été établi pour toutes les revendications

| Lieu de la recherche | Date d'achèvement de la recherche | Examinateur |
|---|---|---|
| La Haye | 06-09-1979 | REMPP |

OEB Form 1503.1  06.78